# FASCICULE DE BREVET EUROPEEN

(11) **EP 1 302 524 B1**
(45) Date de publication et mention de la délivrance du brevet: **14.12.2005**
(21) Numéro de dépôt: 02292441.9
(22) Date de dépôt: 03.10.2002
(51) Int. Cl.: C09K 19/38, A61K 7/043

(54) **Composition de maquillage comprenant un polymère à cristaux liquides**
Make-up Zusammensetzung, die ein flüssigkristallines Polymer enthält
Make-up composition comprising a liquid crystal polymer

(30) Priorité: 10.10.2001 FR 0113034
(43) Date de publication de la demande: 16.04.2003
(73) Titulaire: L'OREAL, 75008 Paris (FR)
(72) Inventeur: Ramin, Roland, 75014 Paris (FR)
(74) Mandataire: Boulard, Denis

(56) Documents cités:
- EP-A- 1 201 726
- DE-A- 19 704 506
- US-A- 6 107 447
- US-B1- 6 207 770

## Description

La présente invention a pour objet une composition cosmétique de maquillage comprenant des particules d'un polymère à cristaux liquides, ainsi qu'un procédé de maquillage des matières kératiniques. La composition selon l'invention peut être appliquée sur les matières kératiniques d'êtres humains, comme les ongles, la peau, les cils, les sourcils, les cheveux. Plus spécialement, l'invention porte sur un vernis à ongles.

La composition de maquillage peut être un vernis à ongles, un fard à joue ou à paupières, un fond de teint, un produit de maquillage pour les lèvres, un produit d'eye-liner, un produit de maquillage du corps, un mascara, un produit de maquillage pour les sourcils. La composition peut également être appliquée sur les accessoires de maquillage comme les faux ongles, faux cils, perruques ou encore sur des pastilles ou des patchs adhérents sur la peau ou les lèvres (du type mouches),

Les compositions de maquillage contiennent de façon connue des matières colorantes pour conférer à la composition la couleur souhaitée.
Les matières colorantes habituellement utilisées sont les pigments organiques tels que les laques ou les pigments minéraux, notamment les nacres.
Pour obtenir des effets de couleurs originaux, il est également connu des documents US-A-5851277 et EP-A-815826 des compositions de maquillage comprenant des pigments interférentiels, en particulier des polyorganosiloxanes comportant des groupements cristaux liquides. Des pigments interférentiels sont également décrits dans les brevets US-A-5362315 et US-A-5851604. Ces pigments interférentiels produisent une couleur dans une gamme de nuance comprise entre aux moins deux couleurs spécifiques et variant en fonction de l'incidence de la lumière et de l'angle d'observation. De tels pigments sont notamment vendus sous la dénomination « HELICONE® » par la société WACKER, appelés également sous le nom CTFA de Polysilicone-12.
Toutefois, on a constaté que ces pigments interférentiels siliconés ne permettent pas d'obtenir un maquillage brillant satisfaisant, ces pigments faisant notamment chuter la brillance des films de vernis à ongles.

La présente invention a donc pour but de proposer une composition de maquillage , en particulier un vernis à ongles, permettant d'obtenir un maquillage brillant présentant un effet de couleur interférentiel.

Le demandeur a constaté qu'il est possible d'obtenir un maquillage présentant de bonne propriété de brillance en utilisant des particules d'un polymère à cristaux liquides particulier.

De façon plus précise, l'invention a pour objet une composition cosmétique, notamment de maquillage, comprenant, dans un milieu cosmétiquement acceptable, des particules d'un polymère à cristaux liquides tel que défini ci-après.

L'invention a également pour objet un procédé cosmétique de maquillage des matières kératiniques, notamment des ongles, comprenant l'application sur les matières kératiniques d'une composition telle que définie précédemment.

L'invention a aussi pour objet l'utilisation de particules d'un polymère à cristaux liquide tel que défini ci-après, dans une composition cosmétique de maquillage, pour obtenir un maquillage brillant déposé sur les matières kératiniques, notamment sur les ongles.

L'invention a aussi pour objet un procédé cosmétique de maquillage des matières kératiniques comprenant l'application sur les matières kératiniques d'une première couche, appelée aussi couche de base, d'une première composition cosmétique comprenant dans un milieu cosmétiquement acceptable au moins une matière colorante, puis l'application sur au moins une partie de ladite première couche, d'une deuxième couche, appelée aussi couche de surface, d'une deuxième composition cosmétique comprenant, dans un milieu cosmétiquement acceptable, des particules de polymère à cristaux liquide tel que défini ci-après,
la première composition ne comprenant pas de particules du polymère à cristaux liquides comme présentes dans la deuxième composition.

L'invention a aussi pour objet un kit de maquillage comprenant :
- une première composition cosmétique (appelée aussi composition de base) comprenant, dans un milieu cosmétiquement acceptable, une première matière colorante, et
- une deuxième composition cosmétique (appelée aussi composition de surface) comprenant, dans un milieu cosmétiquement acceptable, des particules de polymère à cristaux liquides tels que définis ci-après,
la première composition ne comprenant pas de particules du polymère à cristaux liquides comme présentes dans la deuxième composition,
les première et deuxième compositions étant conditionnées dans des récipients distincts.

L'invention a également pour objet un support maquillé comprenant un maquillage susceptible d'être obtenu selon l'un des procédés de maquillage définis précédemment et appliqué sur ledit support, ledit support étant choisi parmi les faux ongles, les faux cils, les postiches, les perruques, les pastilles ou les patchs adhérents sur la peau ou les lèvres.

Le polymère à cristaux liquides présent dans la composition selon l'invention est un polymère susceptible d'être obtenu par polymérisation d'un mélange de monomères comprenant :
- a) au moins un premier monomère A de formule (I) Y¹-A¹-M¹- A²-Y² dans laquelle
   - i) Y¹ et Y² , identiques ou différents, représentent un groupe acrylate ou méthacrylate, de préférence un groupe acrylate ;
   - ii) A¹ et A², identiques ou différents, représentent un groupement de formule -CₙH₂ₙ-, dans laquelle n est un nombre entier allant de 1 à 20, de préférence allant de 2 à 6, et mieux égal à 4 ;
   - iii) M¹ désigne un groupement de formule générale (I') -R¹-X¹-R²-X²-R³-X³-R⁴-,
   dans laquelle R¹ et R⁴ désignent -O-, R² et R³ désignent -COO-.
   et X¹,X²,X³ étant un groupement 1,4 phénylène, le groupe carbonyl -CO- respectivement de R² et de R³ étant lié respectivement au groupement X¹, X³ ,
   et
- b) au moins un deuxième monomère B chiral de formule (II) V¹-W¹-Z-W²-V²,
   dans laquelle
   - i) V¹ désigne un groupement acrylate ou méthacrylate, et de préférence un groupe acrylate, et V² désigne un groupement alkyle en C₁-C₂₀, un alkoxy en C₁-C₂₀, un alkoxy(C₁-C₂₀)carbonyl, -OH, et de préférence désigne un groupement alkoxy en C₁-C₂₀, notamment en C₁-C₄, et en particulier un groupement méthoxy ;
   ii) W¹ représente un groupement divalent de formule X'¹-CO-O-,
   W² représente un groupement divalent de formule -O-CO-X'¹-,
   dans lesquelles X'¹ désigne un groupe 1,4-phénylène,
   et Z désigne un groupement chiral à deux liaisons, issu du groupement dianhydrohexite, en particulier un radical divalent de formule :

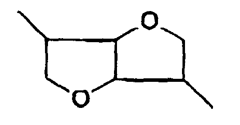

Dans les définitions du premier monomère A décrit précédemment, il est clair que la présence de groupements de deux ou plusieurs atomes oxygénés liés entre eux est exclue.

De préférence, le mélange de monomères comprend de 70 à 99 % en poids de monomère A et de 1 à 30 % en poids de monomère B, par rapport au poids total de monomère A et de monomère B, et mieux comprend de 90 à 95 % en poids de monomère A et de 5 à 10 % en poids de monomère B.

De préférence, la concentration des groupes polymérisables présents dans le mélange de monomère A et de monomère B (groupes polymérisables Y¹, Y² du monomère A et groupes polymérisables V¹, V² du monomère B) va de 3,2 à 15 mmoles/g.

Selon un mode particulier de réalisation de l'invention, le polymère à cristaux liquides est tel que le mélange de monomère A et de monomère B comprend des groupes polymérisables, dont au moins 90 % sont présents dans des monomères ayant au moins deux groupes polymérisables, en une concentration allant de 3,2 et 15 mmoles/g.

Préférentiellement, le polymère à cristaux liquide comprend essentiellment ou consiste en un mélange des monomères A et B définis précédemment.

Avantageusement, le polymère à cristaux liquide présente un pas d'hélice supérieur à 450 nm, notamment allant de 455 nm à 5000 nm, de préférence allant de 455 nm à 1000 nm, et mieux allant de 455 nm à 650 nm.

Le monomère A a de préférence un poids moléculaire moyen en poids allant de 150 à 800, et de préférence allant de 460 à 625. Le monomère A est avantageusement un dérivé de dibenzoate d'hydroquinone non substitué.

Le monomère B peut avoir un poids moléculaire moyen en poids allant de 500 à 1000, et de préférence allant de 500 à 700.

Le polymère à cristaux liquides défini précédemment peut être préparé suivant les procédés connus dans l'état de la technique, tels que ceux décrits dans les documents US 5362315, US 5807497, à partir du mélange de monomère décrit précédemment

Par exemple, on applique un mélange des monomères A et B définis précédemment sur un fond lisse et qu'on oriente, on réticule de façon tridimensionnelle par une polymérisation du mélange de monomères et on désolidarise du fond.

Le mélange de monomères est appliqué de préférence en une épaisseur de 3 à 15 µm, et plus particulièrement de 3 à 6 µm sur la surface lisse.

L'orientation peut par exemple s'effectuer par cisaillement, en utilisant une racle ou un rouleau.

La polymérisation du mélange de monomères orienté peut, de manière déjà connue, se faire par exemple de façon radicalaire avec utilisation d'initiateurs thermiques du commerce, en utilisant des rayons d'électrons ou de la lumière UV en combinaison avec des photoinitiateurs du commerce, ou bien par des réac tions d'addition ou de condensation.

La réticulation des mélanges de monomères, dans l'état structurel chiral, se fait de préférence au moyen d'une polyréaction qui, selon le type des groupes polymérisables, polycondensables ou polyadditionnables, se déroule sous la forme d'une polymérisation radicalaire, ionique ou catalysée au métal, ou d'une réaction de polycondensation ou d'une réaction de polyaddition.

L'initiation de la polymérisation radicalaire peut s'effectuer au moyen d'initiateurs correspondants ou par un rayonnement par UV, en utilisant des photoinitiateurs du commerce ou par un rayonnement à haute énergie, tel qu'un rayonnement d'électrons. Un avantage de la polymérisation thermique des radicaux ou de la polymérisation via un durcissement aux rayons d'électrons réside dans le fait qu'au mélange polymérisable peut également être ajouté un agent de protection contre la lumière, tel qu'un absorbeur d'UV (UVA) ou des capteurs de radicaux (HALS), pour stabiliser les pigments ou les films résultants face à la lumière UV, par exemple pour des applications extérieures, sans entraîner des pertes au niveau de la conversion de polymérisation, tel que ceci est le cas lors du durcissement aux UV, du fait de l'effet d'écrantage du photoinitiateur par un UVA. Il n'y a ainsi aucune diminution de la densité de réticulation.

Si le durcissement des films LC se fait de façon peroxydique ou par un rayonnement d'électrons, le mélange de monomères contient de préférence des agents de protection contre la lumière, du commerce, tels que des absorbeurs à UV ou des capteurs de radicaux, en une concentration globale de 0,5 à 5 % en poids.

Outre les photostabilisateurs, les mélanges de monomères peuvent également contenir d'autres additifs usuels visant à inhiber l'oxydation, à inhiber la polymérisation, ou des additifs visant à améliorer les propriétés rhéologiques. De plus, les charges absorbantes, telles que les pigments ou la suie, ainsi que des colorants ou des pigments à fluorescence peuvent être contenus.

Le film obtenu après la polymérisation est ensuite broyé en particules, notamment sous forme de plaquettes.
De préférence, les particules de polymère à cristaux liquides ont une plus grande taille allant de 1 µm à 3 mm, et de préférence allant de 30 µm à 500 µm. Ces particules sont avantageusement en forme de plaquettes.
Les particules peuvent être séparées (triées) par un procédé à sélectivité de la taille de grains.

De tels polymères et leurs particules sont décrits dans la demande EP-A-1046692.

Comme particules de polymère à cristaux liquides, on peut utiliser celles connues sous le nom CTFA Polyacrylate-4 et vendues sous les dénominations « HELICONE® HC Sapphire », « HELICONE® HC Scarabeus », « HELICONE® HC Jade », « HELICONE® HC Maple », « HELICONE® HC XL Sapphire », « HELICONE® HC XL Scarabeus », « HELICONE® HC XL Jade », « HELICONE® HC XL Maple » par la société WACKER.

Les particules du polymère à cristaux liquide peuvent être présentes dans la composition selon l'invention en une teneur allant de 0,01 % à 99 % en poids, par rapport au poids total de la composition, avantageusement allant de 0,1 % à 60 % en poids, de préférence allant de 1 % à 30 % en poids, et mieux allant de 5 % à 15 % en poids.

La composition selon l'invention, ou la composition de base et/ou de surface, peut comprendre un milieu cosmétique hydrophile ou un milieu lipophile.

La composition de l'invention, ou la composition de base et/ou de surface, peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La phase hydrophile peut, en outre, contenir des éthers en C₂ et des aldéhydes en C₂-C₄ hydrophiles. L'eau ou le mélange d'eau et de solvants organiques hydrophiles peut être présent dans la composition selon l'invention, ou la composition de base et/ou de surface, en une teneur allant de 0 % à 90 % (notamment 0,1 % à 90 %) en poids, par rapport au poids total de la composition, et de préférence de 0 % à 60 % en poids (notament 0,1 % à 60 % en poids). En particulier, la composition peut comprendre une phase continue hydrophile.

La composition selon l'invention, ou la composition de base et/ou de surface, peut comprendre une phase grasse, notamment constituée de corps gras liquides à température ambiante (25°C en général) et/ou de corps gras solides à température ambiante tels que les cires, les corps gras pâteux, les gommes et leurs mélanges. Cette phase grasse peut, en outre, contenir des solvants organiques lipophiles. La phase grasse peut former la phase continue de la composition selon l'invention, ou de la composition de base et/ou de surface. La composition peut être anhydre.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

La composition selon l'invention, ou la composition de base et/ou de surface, peut comprendre un ou plusieurs solvants organiques, cosmétiquement acceptables (tolérance, toxicologie et toucher acceptables). Ces solvants peuvent être présents en une teneur allant de 0,1 à 90 % et mieux de 0,1 à 60% en poids, par rapport au poids total de la composition et mieux de 0,1 à 30 %.

Comme solvants utilisables dans la composition de l'invention, ou dans la composition de base et/ou de surface, on peut citer:
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

Ces solvants organiques conviennent plus particulièrement pour le maquillage des ongles : la composition constitue alors un vernis à ongles.

Le solvant organique peut être présent dans la composition selon l'invention, ou la composition de base et/ou de surface, en une teneur allant de 30 à 99 % en poids, par rapport au poids total de chaque composition, et de préférence de 60 % à 90 % en poids.

La composition de l'invention, ou la composition de base et/ou de surface, peut en outre comprendre, avantageusement un corps gras solide ou pâteux à température ambiante, comme les gommes ou les cires.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C. Comme cire utilisable dans la composition de l'invention, on peut citer la cire d'abeilles, la cire de Carnauba, la cire de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50 % en poids de cires, par rapport au poids total de la composition et mieux de 1 à 30 % en poids.

La composition selon l'invention, ou la composition de base et/ou de surface, peut en outre comprendre un polymère filmogène. Dans la présente demande, on entend par "polymère filmagène". un polymère apte à former à lui seul ou en présence d'un agent auxiliaire de filmification, un film continu et adhérent sur un support, notamment sur les matières kératiniques.

Le polymère filmogène peut être choisi parmi les polymères radicalaires, les polycondensats ou les polymères d'origine naturelle. Le polymère filmogène peut être dissous ou dispersé sous forme de particules solides dans le milieu cosmétiquement acceptable de la composition. En particulier, le polymère peut se présenter sous forme de particules solides en dispersion aqueuse.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaires peuvent être notamment des polymères, ou des copolymères, vinyliques, notamment des polymères acryliques.

Les polymères filmogènes vinyliques peuvent également résulter de l'homopolymérisation ou de la copolymérisation d'au moins un monomère choisi parmi les esters vinyliques et les monomères styrèniques.

Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Parmi les polycondensats filmogènes, on peut citer les polyuréthanes, les polyesters, les polyesters amides, les polyesters à chaîne grasse, les polyamides, et les résines époxyesters, les résines résultant de la condensation de formaldéhyde avec une arylsulfonamide, les résines aryl-sulfonamide époxy.

Les polymères d'origine naturelle, éventuellement modifiés, peuvent être choisis parmi la résine shellac, la gomme de sandaraque, les dammars, les élémis, les copals, les polymères cellulosiques tels que la nitrocellulose, l'acétate de cellulose, l'acétobutyrate de cellulose, l'acétopropionate de cellulose, l'éthyl cellulose, et leurs mélanges.

Comme polymère filmogène, on peut utiliser en particulier les polymères acryliques, les polyuréthanes, les polyesters, les polyamides, les polyurées, les polymères cellulosiques.

Le polymère filmogène peut être présent dans la composition selon l'invention, notamment la composition de base et/ou de surface, en une teneur en matières sèches de polymère allant de 0,1 % à 60 % en poids par rapport au poids total de la composition, de préférence de 0,5 % à 40 % en poids, et mieux de 1 % à 30 % en poids.

Pour améliorer les propriétés filmogènes de la composition, un agent auxiliaire de filmification peut être présent dans la composition.

Un tel agent auxiliaire de filmification peut être choisi parmi tous les composés connus de l'homme du métier comme étant susceptibles de remplir la fonction recherchée, et être notamment choisi parmi les agents plastifiants.

En outre, lorsque la composition selon l'invention comprend un polymère filmogène sous forme de particules dispersées dans le milieu correspondant de la composition, l'agent auxiliaire de filmification peut aussi être choisi parmi les agents de coalescence.

La composition selon l'invention, ou la composition de base et/ou de surface, peut en outre comprendre des matières colorantes additionnelles, différentes des particules de polymère à cristaux liquides décrites précédemment. La matière colorante peut notamment être choisie parmi les colorants (hydrosolubles ou liposolubles) ou les matières colorantes pulvérulentes tels que les pigments, les nacres, les paillettes bien connues de l'homme du métier. Les matières colorantes additionnelles peuvent être présentes dans la composition, notamment dans la composition de base et/ou de surface, en une teneur allant de 0,01 % à 25 % en poids, par rapport au poids de chaque composition. Selon un mode particulier de réalisation de l'invention, la composition de base peut être de couleur noire.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être présents dans la composition, notamment dans la composition de base et/ou de surface, à raison de 0 à 15 % (notamment 0,01 % à 15 %) par rapport au poids de la composition, de préférence de 0,01 % à 10 % en poids, et mieux de 0,02 % à 5 % en poids.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les nacres peuvent être présentes dans la composition, notamment dans la composition de base et/ou de surface, à raison de 0 à 25 % (notamment 0,01 % à 25 %) en poids, par rapport au poids total de la composition, de préférence de 0,01 % à 15 % en poids, et mieux de 0,02 % à 5 % en poids.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants liposolubles sont par exemple l'huile de soja, le brun Soudan, le DC Yellow 11, le DC orange 5, le jaune quinoléine, le Rouge Soudan III (nom CTFA D&C red 17), la lutéine, le vert de quinizarine (nom CTFA DC green 6), le pourpre d'Alizurol SS (nom CTFA DC violet N°2), les dérivés caroténoïdes comme le lycopène, le béta-carotène, la bixine, la capsantéine, et/ou leurs mélanges.

La composition selon l'invention, notamment la composition de base et/ou de surface, peut comprendre en outre des charges. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Coming) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention, notamment la composition de base et/ou de surface, peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 s⁻¹, après 10 minutes de mesure en géométrie cône/plan). La composition peut être à phase continue organique, notamment anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention, notamment dans la composition de base et/ou de surface, peut également contenir des ingrédients couramment utilisés en cosmétique, tels que les vitamines, les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition de l'invention peut être obtenue selon les procédés de préparation classiquement utilisés en cosmétique.

Les exemples de compositions ci-après sont donnés à titre illustratif et sans caractère limitatif.

### Exemple 1 :

On a préparé un vernis à ongles ayant la composition suivante :
- Nitrocellulose 10 g
- Plastifiants et résines 15 g
- Agent épaississant 1,5 g
- Particules de polymère LC vendues sous la dénomination « HELICONE® HC Maple » par la société Wacker 3 g
- Alcool isopropylique 5 g
- Acétate d'éthyle 20 g
- Acétate de butyle qsp 100 g

Aprés application de la composition sur les ongles, on a obtenu un film de maquillage présentant un aspect changeant très brillant.

### Exemple 2 :

On a préparé un vernis à ongles ayant la composition suivante :
- Nitrocellulose 10 g
- Plastifiants et résines 15 g
- Agent épaississant 1,5 g
- Particules de polymère LC vendues sous la dénomination « HELICONE® HC Scarabeus XL » par la société Wacker 10 g
- Alcool isopropylique 5 g
- Acétate d'éthyle 20 g
- Acétate de butyle qsp 100 g

Après application de la composition sur les ongles, on a obtenu un film de maquillage de couleur rouge présentant un aspect changeant très brillant.

### Exemples 3 et 4 comparatifs :

On a préparé un vernis à ongles selon l'invention (exemple 3) et un vernis à ongles ne faisant pas partie de l'invention (exemple 4) ayant la composition suivante (les teneurs sont exprimées en gramme) :

| **Ingrédient** | **Exemple 3** | **Exemple 4** |
|---|---|---|
| Nitrocellulose | 11 | 11 |
| Résine tosylamlde/formaldéhyde | 10 | 10 |
| Acetyl citrate de tributyle | 5.7 | 5.7 |
| bentonite | 1.4 | 1.4 |
| Alcool isopropylique | 5.5 | 5.5 |
| Pigment rouge | 0.014 | 0.07 |
| Particules de polymère LC (Hélicone® HC Maple XL de Wacker) | 5 | 0 |
| Pigment nacrant (Mica-oxyde de titane- oxyde de fer brun) | 0 | 0.6 |
| Acétate d'Ethyle | 31.5 | 31.5 |
| Acétate de butyle | qsp 100 | qsp 100 |

Après application de chaque vernis sur les ongles, on a constaté que le vernis à ongles de l'exemple 3 selon l'invention permet d'obtenir un maquillage bien plus brillant que celui obtenu avec le vernis de l'exemple 4 qui ne contient pas de particules de polymères à cristaux liquides.

### Exemples 5 et 6 comparatifs :

On a préparé un vernis à ongles selon l'invention (exemple 5) et un vernis à ongles ne faisant pas partie de l'invention (exemple 6) ayant la composition suivante (les teneurs sont exprimées en gramme):

| **Ingrédient** | **Exemple 5** | **Exemple 6** |
|---|---|---|
| Nitrocellulose | 10.6 | 10.6 |
| Résine tosylamide/formaldéhyde | 8 | 8 |
| Acetyl citrate de tributyle | 5.4 | 5.4 |
| Bentonite | 1 | 1 |
| Silice pyrogénée | 1.5 | 1.5 |
| Mica-oxyde de fer brun (Cloisonné Sparkle Rouge 450J de la société Engelhard) | 2 | 2 |
| Particules de polymère LC (Hélicone® HC Jade XL de Wacker) | 1.61 | 0 |
| Pigment interférentiel Polysilicone-12 (Heticone® 8575 de Wacker) | 0 | 1.61 |
| Alcool isopropylique | 4.9 | 4.9 |
| Acétate d'Ethyle | 26-9 | 26.9 |
| Acétate de Butyle | qsp 100 | qsp 100 |

La brillance de chaque vernis à ongles a été mesurée pour un film humide de 300 µm d'épaisseur déposé sur carte de contraste type LENETA Form 1A-PENOPAC puis séché pendant 24 heures. La mesure a été effectuée à l'aide d'un brillance-mètre de type micro-TRI-gloss de Byk Gardner sous un angle de faisceau lumineux de 60°. On a obtenu les résultats suivants :
Exemple 5 : 58,8
Exemple 6 : 44

On a constaté que le vernis de l'exemple 5 selon l'invention permet d'obtenir un maquillage plus brillant que celui obtenu avec le vernis de l'exemple 6 ne faisant pas partie de l'invention.

### Exemple 7 :

On a préparé 2 compositions A et B de vernis à ongles suivantes (teneurs exprimées en gramme) :

| | Composition A Base Coat | Composition B Top Coat |
|---|---|---|
| Nitrocellulose | 10 | 10 |
| Plastifiants et résines | 5 | 5 |
| Agent épaississant | 1.5 | 1.5 |
| Oxyde de fer noir | 3 | 0g |
| Particules de polymère LC (Hélicone® HC Sapphire de Wacker) | 0 | 0.5 |
| Particules de polymère LC (Hélicone® HC Scarabeus de Wacker) | 0 | 1.5 |
| Alcool Isopropylique | 5 | 5 |
| Acetate d'Ethyle | 20 | 20 |
| Acétate de Butyle | qsp 100 | qsp 100 |

On a appliqué sur l'ongle une couche de base de la composition A puis après séchage une couche de surface de la composition B. On a obtenu un maquillage brillant présentant un effet interférentiel sur un fond noir.

### Exemple 8 :

On a préparé une poudre compacte ayant la composition suivante :
- Oxyde de fer jaune 1 g
- Oxyde de fer brun 0,55 g
- Carbonate de calcium hydraté 2 g
- Polydiméthylsiloxne (DOW CORNING fluid 200 - 10 cst de DOW CORNING) 6,8 g
- Mélange de résine triméthylsiloxysilicate et de polydiméthylsiloxane (33/67) (DOW CORNING 593 Fluid de Dow Coming) 2 g
- Polyméthycetyl diméthylsiloxane (ABIL WAX 9801 de Goldschmidt) 1,2 g
- Mica 30 g
- Particules de polymère LC vendues sous la dénomination « HELICONE® HC Scarabeus XL » par la société Wacker 5 g
- Talc qsp 100 g

Le mélange pulvérulent est compacté dans une coupelle. On obtient ainsi une poudre cosmétique compacte qui peut être utilisée comme fard à paupière ou fard à joue conférent un maquillage brillant.

### Exemple 9 :

On a préparé un stick de rouge à lèvres ayant la composition suivante :
- Huile de ricin 72 g
- Stéarate d'octacosanyle 8 g
- Particules de polymère LC vendues sous la dénomination « HELICONE® HC Maple » par la société Wacker 10 g

Après application sur les lèvres, on obtient un maquillage très brillant.

## Revendications

1. Composition cosmétique comprenant, dans un milieu cosmétiquement acceptable, des particules de polymère à cristaux liquides susceptible d'être obtenu par polymérisation d'un mélange de monomères comprenant :
- a) au moins un premier monomère A de formule (I) Y¹-A¹ -M¹- A²-Y² dans laquelle
- i) Y¹ et Y², identiques ou différents, représentent un groupe acrylate ou méthacrylate,
- ii) A¹ et A², identiques ou différents, représentent un groupement de formule -CₙH₂ₙ-, dans laquelle n est un nombre entier allant de 1 à 20 ;
- iii) M¹ désigne un groupement de formule générale (I') -R¹-X¹-R²-X²-R³-X³-R⁴-,
dans laquelle R¹ et R⁴ désignent -O-, R² et R³ désignent -COO-,
et X¹,X²,X³ étant un groupement 1,4-phénylène, le groupe carbonyl -CO- respectivement de R² et de R³ étant lié respectivement au groupement X¹, X³,
et
- b) au moins un deuxième monomère B chiral de formule (II) V¹-W¹-Z-W²-V²,
dans laquelle
- i) V¹ désigne un groupement acrylate ou méthacrylate et V² désigne un groupement alkyle en C₁-C₂₀, un alkoxy en C₁-C₂₀, un alkoxy(C₁-C₂₀)carbonyl, -OH,
- ii) W¹ représente un groupement divalent de formule)-X¹-CO-O-,
W² représente un groupement divalent de formule -O-CO-X'¹-,
dans lesquelles X^{'1} désigne un groupe 1,4-phénylène,
et Z désigne un groupement chiral à deux liaisons, issu du groupement dianhydrohexite.

2. Composition selon la revendication 1, **caractérisée par le fait que** V¹ représente un groupe acrylate.

3. Composition selon la revendication 1 ou 2, **caractérisée par le fait que** V² représente un groupement alkoxy en C₁-C₂₀.

4. Composition selon l'une des revendications 1 à 3, **caractérisée par le fait que** Y¹ et Y² représentent un groupe acrylate.

5. Composition selon l'une des revendications 1 à 4, **caractérisée par le fait que** A¹ et A², identiques ou différents, représentent un groupement de formule -CₙH₂ₙ-, dans laquelle n est un nombre entier allant de 2 à 6.

6. Composition selon la revendication 5, **caractérisée par le fait que** n est égal à 4.

7. Composition selon l'une quelconque des revendications 1 à 6, **caractérisée par le fait que** V¹ désigne un groupement acrylate et V² désigne un groupement alkoxy en C₁-C₄.

8. Composition selon la revendication 7, **caractérisée par le fait que** V² désigne un groupement méthoxy.

9. Composition selon l'une quelconque des revendications 1 à 8, **caractérisée par le fait que** Z désigne un radical divalent de formule :

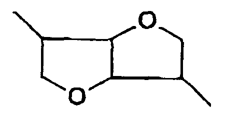

10. Composition selon l'une quelconque des revendications précédentes, **caractérisée par le fait que** le mélange de monomères comprend de 70 à 99 % en poids de monomère A et de 1 à 30 % en poids de monomère B, par rapport au poids total de monomère A et de monomère B.

11. Composition selon l'une quelconque des revendications précédentes, **caractérisée par le fait que** le mélange de monomères comprend de 90 à 95 % en poids de monomère A et de 5 à 10 % en poids de monomère B, par rapport au poids total de monomère A et de monomère B.

12. Composition selon l'une quelconque des revendications précédentes, **caractérisée par le fait que** le polymère à cristaux liquides présente un pas d'hélice supérieur à 450 nm.

13. Composition selon la revendications 12, **caractérisée par le fait que** le polymère à cristaux liquides présente un pas d'hélice allant de 455 nm à 5000 nm.

14. , Composition selon la revendication 12 ou 13, **caractérisée par le fait que** le polymère présente un pas d'hélice allant de 455 à 1 000 nm.

15. Composition selon l'une quelconque des revendications précédentes, **caractérisée par le fait que** le mélange de monomère A et de monomère B comprend des groupes polymérisables, dont au moins 90 % sont présents dans des monomères ayant au moins deux groupes polymérisables, en une concentration allant de 3,2 à 15 mmoles/g.

16. Composition selon l'une quelconque des revendications précédentes, **caractérisée par le fait que** le monomère A a un poids moléculaire moyen en poids allant de 150 à 800.

17. Composition selon la revendication 16 **caractérisée en ce que** le monomère a un poids moléculaire moyen en poids allant de 460 à 625.

18. Composition selon l'une quelconque des revendications précédentes, **caractérisée par le fait que** le monomère B a un poids moléculaire moyen en poids allant de 500 à 1000.

19. Composition selon la revendication précédente, **caractérisée par le fait que** le monomère B a un poids moléculaire moyen en poids allant de 500 à 700.

20. Composition selon l'une quelconque des revendications précédentes, **caractérisée par le fait que** les particules de polymère à cristaux liquides sont sous la forme de plaquettes.

21. Composition selon l'une quelconque des revendications précédentes, **caractérisée par le fait que** les particules de polymères à cristaux liquides ont une plus grande taille allant de 1 µm à 3 mm.

22. Composition selon la revendication 21, **caractérisée en ce que** les particules de polymères à cristaux liquides ont une plus grande taille de 30 µm à 500 µm.

23. Composition selon l'une quelconque des revendications précédentes, **caractérisée par le fait que** les particules de polymère à cristaux liquides sont présentes en une teneur allant de 0,1 % à 99 % en poids, par rapport au poids total de la composition.

24. Composition selon la revendication 23, **caractérisée par le fait que** les particules de polymère à cristaux liquides sont présentes en une teneur allant de 0,1 % à 60 % en poids.

25. Composition selon la revendication 24, **caractérisée par le fait que** les particules de polymère à cristaux liquides sont présentes en une teneur allant de 1 à 30 % en poids par rapport au poids total de la composition.

26. Composition selon la revendication 25, **caractérisée par le fait que** les particules de polymère à cristaux liquides sont présentes en une teneur allant de 5 à 15 % en poids total de la composition.

27. Composition selon l'une quelconque des revendications précédentes, **caractérisée par le fait que** la composition comprend un milieu cosmétique hydrophile ou lipophile.

28. Composition selon l'une quelconque des revendications précédentes, **caractérisée par le fait qu'**elle comprend de l'eau ou un mélange d'eau et de solvant ganique hydrophile.

29. Composition selon l'une quelconque des revendications précédentes, **caractérisée par le fait qu'**elle comprend une phase grasse.

30. Composition selon la revendication 29, **caractérisée par le fait que** la composition contient un ingrédient choisi parmi les huiles, les cires, les corps gras pâteux, les gommes, et leurs mélanges.

31. Composition selon l'une quelconque des revendications précédentes, **caractérisée par le fait qu'**elle contient un solvant organique.

32. Composition selon l'une quelconque des revendications précédentes, **caractérisée par le fait qu'**elle comprend un polymère filmogène.

33. Composition selon la revendication 32, **caractérisée par le fait que** le polymère filmogène est choisi dans le groupe formé par les polymères vinyliques, les polyuréthanes, les polyesters, les polyamides, les polyurées, les polymères cellulosiques.

34. Composition selon l'une des revendications 32 ou 33, **caractérisée par le fait que** le polymère filmogène est présent en une teneur en matières sèches de polymère allant de 0,1 % à 60 % en poids par rapport au poids total de la composition.

35. Composition selon l'une quelconque des revendications précédentes, **caractérisée par le fait qu'**elle comprend une matière colorante additionnelle différente des particules dudit polymère à cristaux liquides.

36. Composition selon la revendication 35, **caractérisée par le fait que** la matière colorante additionnelle est choisie dans le groupe formé par les pigments, les nacres, les colorants hydrosolubles ou liposolubles.

37. Composition selon l'une des revendications 35 ou 36, **caractérisée par le fait que** la matière colorante additionnelle est un pigment choisi parmi le dioxyde de titane, les oxydes de zirconium, les oxydes de cérium, les oxydes de zinc, les oxydes de fer, les oxydes de chrome, le violet de manganèse, le bleu outremer, hydrate de chrome, le bleu ferrique, la poudre d'aluminium, la poudre de cuivre, le noir de carbone, les pigments de type D & C, et les laques à base de carmin de cochenille, de baryum, strontium, calcium, aluminium.

38. Composition selon l'une des revendications 36 ou 37, **caractérisée par le fait que** les pigments sont présents en une teneur allant de 0,01 % à 15 % en poids, par rapport au poids total de la composition,

39. Composition selon l'une des revendications 36 à 38, **caractérisée par le fait que** les pigments sont présents en une teneur allant de 0,02 % à 5 % en poids par rapport au poids total de la composition.

40. Composition selon l'une quelconque des revendications 36 à 39, **caractérisée par le fait que** la matière colorante additionnelle est une nacre choisie parmi le mica recouvert de titane ou d'oxychlorure de bismuth, le mica titane recouvert avec des oxydes de fer, le mica titane recouvert avec du bleu ferrique ou de l'oxyde de chrome, le mica titane recouvert avec un pigment organique, les pigments nacrés à base d'oxychlorure de bismuth.

41. Composition selon l'une des revendications 36 ou 40, **caractérisée par le fait que** les nacres sont présentes en une teneur allant de 0,01 % à 25 % en poids, par rapport au poids de la composition.

42. Composition selon l'une quelconque des revendications précédentes, **caractérisée par le fait qu'**elle comprend un ingrédient cosmétique choisi dans le groupe formé par les charges, les vitamines, les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

43. Composition selon l'une des revendications précédentes, **caractérisée par le fait qu'**elle se présente sous la forme d'un vernis à ongles, de mascara, d'eye-liner, de produit de maquillage pour les lèvres, de fond de teint, de fard à joues ou à paupières, de produit pour les sourcils, de produit de maquillage du corps.

44. Composition selon l'une quelconque des revendications précédentes, **caractérisée par le fait qu'**elle se présente sous la forme de vernis à ongles.

45. Procédé cosmétique de maquillage des matières kératiniques, **caractérisé par le fait que** l'on applique sur les matières kératiniques une composition selon l'une quelconque des revendications 1 à 44.

46. Procédé cosmétique de maquillage des matières kératiniques comprenant l'application sur les matières kératiniques d'une première couche, appelée aussi couche de base, d'une première composition cosmétique comprenant dans un milieu cosmétiquement acceptable au moins une matière colorante, puis l'application sur au moins une partie de ladite première couche, d'une deuxième couche d'une deuxième composition cosmétique conforme à la composition telle que définie selon l'une quelconque des revendications 1 à 44,
la première composition ne comprenant pas de particules du polymère à cristaux liquides comme présentes dans la deuxième composition.

47. Procédé selon la revendication 46, **caractérisé par le fait que** la première composition comprend une matière colorante choisie parmi les pigments, les nacres, les colorants hydrosolubles ou liposolubles.

48. Procédé selon la revendication 46 ou 47, **caractérisé par le fait que** la première composition comprend un polymère filmogène.

49. Procédé selon l'une quelconque des revendications 46 à 48, **caractérisé par le fait que** la première composition comprend un ingrédient cosmétique choisi dans le groupe formé par les charges, les vitamines, les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

50. Procédé selon l'une quelconque des revendications 48 à 49, **caractérisé par le fait que** la première composition se présente sous la forme d'un vernis à ongles, de mascara, d'eye-liner, de produit de maquillage pour les lèvres, de fond de teint, de fard à joues ou à paupières, de produit pour les sourcils, de produit de maquillage du corps.

51. Procédé selon l'une quelconque des revendications 46 à 50, **caractérisé par le fait que** la première composition se présente sous la forme de vernis à ongles.

52. Kit de maquillage comprenant :
- une première composition cosmétique comprenant, dans un milieu cosmétiquement acceptable, une première matière colorante, et
- une deuxième composition cosmétique conforme à la composition telle que définie selon l'une quelconque des revendications 1 à 44,
la première composition ne comprenant pas de particules du polymère à cristaux liquides comme présentes dans la deuxième composition,
les première et deuxième compositions étant conditionnées dans des récipients distincts.

53. Kit de maquillage selon la revendication 52, **caractérisé par le fait que** la première composition comprend une matière colorante choisi parmi les pigments, les nacres, les colorants hydrosolubles ou liposolubles.

54. Kit de maquillage selon la revendication 52 ou 53, **caractérisé par le fait que** la première composition comprend un polymère filmogène.

55. Kit de maquillage selon l'une quelconque des revendication 52 à 54, **caractérisé par le fait que** la première composition comprend un ingrédient cosmétique choisi dans le groupe formé par les charges, les vitamines, les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

56. Kit de maquillage selon l'une quelconque des revendications 52 à 55, **caractérisé par le fait que** la première composition se présente sous la forme d'un vernis à ongles, de mascara, d'eye-liner, de produit de maquillage pour les lèvres, de fond de teint, de fard à joues ou à paupières, de produit pour les sourcils, de produit de maquillage du corps.

57. Kit de maquillage selon l'une quelconque des revendications 52 à 56, **caractérisé par le fait que** la première composition se présente sous la forme de vernis à ongles.

58. Support maquillé comprenant un maquillage susceptible d'être obtenu selon le procédé de maquillage conforme à l'une quelconque des revendications 45 à 51 et appliqué sur ledit support, ledit support étant choisi parmi les faux ongles, les faux cils, les postiches, les perruques, les pastilles ou les patchs adhérents sur la peau ou les lèvres.

## Patentansprüche

1. Kosmetische Zusammensetzung, die in einem kosmetisch akzeptablen Träger flüssigkristalline Polymerpartikel enthält, die durch Polymerisation eines Monomerengemisches erhältlich sind, das enthält:
- a) mindestens ein erstes Monomer A der Formel (I) Y¹-A¹-M¹-A²-Y², worin bedeuten:
- i) Y¹ und Y², die gleich oder verschieden sind, eine Acrylatgruppe oder Methacrylatgruppe;
- ii) A¹ und A², die gleich oder verschieden sind, eine Gruppe der Formel -CₙH₂ₙ-, wobei n eine ganze Zahl im Bereich von 1 bis 20 bedeutet;
- iii) M¹ eine Gruppe der allgemeinen Formel (I') -R¹-X¹-R²-X²-R²-X³-R⁴-, wobei R¹ und R⁴ -O-, R² und R³ -COO- und X¹, X² und X³ eine 1,4-Phenylengruppe bedeuten, wobei die Carbonylgruppe -CO- von R² bzw. R³ an die Gruppe X¹ bzw. X³ gebunden ist, und
- b) mindestens ein zweites chirales Monomer B der Formel (II) V¹-W¹-Z-W²-V², worin bedeuten:
- i) V¹ eine Acrylat- oder Methacrylatgruppe und V² eine C₁-₂₀-Alkylgruppe, eine C₁-₂₀-Alkoxygruppe, eine Alkoxy(C₁₋₂₀)carbonylgruppe, -OH;
- ii) W¹ eine zweiwertige Gruppe der Formel -X'¹-CO-O-, W² eine zweiwertige Gruppe der Formel -O-CO-X'¹, wobei X'¹ eine 1,4-Phenylengruppe bedeutet, und
Z eine chirale Gruppe mit zwei Bindungen, die von der Dianhydrohexitgruppe abgeleitet ist.

2. Zusammensetzung nach Anspruch 1, **dadurch gekennzeichnet, dass** V¹ ein Acrylatgruppe bedeutet.

3. Zusammensetzung nach Anspruch 1 oder 2, **dadurch gekennzeichnet, dass** V² eine C₁₋₂₀-Alkoxygruppe bedeutet.

4. Zusammensetzung nach einem der Ansprüche 1 bis 3, **dadurch gekennzeichnet, dass** Y¹ und Y² eine Acrylatgruppe bedeuten.

5. Zusammensetzung nach einem der Ansprüche 1 bis 4, **dadurch gekennzeichnet, dass** A¹ und A², die gleich oder verschieden sind, eine Gruppe der Formel -CₙH₂ₙ- bedeuten, wobei n eine ganze Zahl von 2 bis 6 ist.

6. Zusammensetzung nach Anspruch 5, **dadurch gekennzeichnet, dass** n 4 ist.

7. Zusammensetzung nach einem der Ansprüche 1 bis 6, **dadurch gekennzeichnet, dass** V¹ eine Acrylatgruppe und V² eine C₁₋₄-Alkoxygruppe bedeutet.

8. Zusammensetzung nach Anspruch 7, **dadurch gekennzeichnet, dass** V² eine Methoxygruppe bedeutet.

9. Zusammensetzung nach einem der Ansprüche 1 bis 8, **dadurch gekennzeichnet, dass** Z eine zweiwertige Gruppe der folgenden Formel bedeutet:

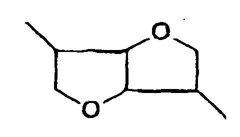

10. Zusammensetzung nach einem der vorhergehenden Ansprüche, **dadurch gekennzeichnet, dass** das Monomerengemisch 70 bis 99 Gew.-% Monomer A und 1 bis 30 Gew.-% Monomer B, bezogen auf das Gesamtgewicht von Monomer A und Monomer B, enthält.

11. Zusammensetzung nach einem der vorhergehenden Ansprüche, **dadurch gekennzeichnet, dass** das Monomerengemisch 90 bis 95 Gew.-% Monomer A und 5 bis 10 Gew.-% Monomer B, bezogen auf das Gesamtgewicht von Monomer A und Monomer B, enthält.

12. Zusammensetzung nach einem der vorhergehenden Ansprüche, **dadurch gekennzeichnet, dass** das flüssigkristalline Polymer eine Ganghöhe über 450 nm aufweist.

13. Zusammensetzung nach Anspruch 12, **dadurch gekennzeichnet, dass** das flüssigkristalline Polymer eine Ganghöhe von 455 bis 5 000 nm aufweist.

14. Zusammensetzung nach Anspruch 12 oder 13, **dadurch gekennzeichnet, dass** das Polymer eine Ganghöhe von 455 bis 1 000 nm besitzt.

15. Zusammensetzung nach einem der vorhergehenden Ansprüche, **dadurch gekennzeichnet, dass** das Gemisch aus Monomer A und Monomer B polymerisierbare Gruppen, von denen mindestens 90 % in Monomeren mit mindestens zwei polymerisierbaren Gruppen vorliegen, in einer Konzentration von 3,2 bis 15 mmol/g enthält.

16. Zusammensetzung nach einem der vorhergehenden Ansprüche, **dadurch gekennzeichnet, dass** das Monomer A eine gewichtsmittlere Molmasse von 150 bis 800 aufweist.

17. Zusammensetzung nach Anspruch 16, **dadurch gekennzeichnet, dass** das Monomer eine gewichtsmittlere Molmasse von 460 bis 625 besitzt.

18. Zusammensetzung nach einem der vorhergehenden Ansprüche, **dadurch gekennzeichnet, dass** das Monomer B eine gewichtsmittlere Molmasse von 500 bis 1 000 aufweist.

19. Zusammensetzung nach dem vorhergehenden Anspruch, **dadurch gekennzeichnet, dass** das Monomer B eine gewichtsmittlere Molmasse von 500 bis 700 aufweist.

20. Zusammensetzung nach einem der vorhergehenden Ansprüche, **dadurch gekennzeichnet, dass** die flüssigkristallinen Polymerpartikel in Form von Plättchen vorliegen.

21. Zusammensetzung nach einem der vorhergehenden Ansprüche, **dadurch gekennzeichnet, dass** die flüssigkristallinen Polymerpartikel eine größere Abmessung von 1 µm bis 3 mm besitzen.

22. Zusammensetzung nach Anspruch 21, **dadurch gekennzeichnet, dass** die flüssigkristallinen Polymerpartikel eine größere Abmessung von 30 bis 500 µm aufweisen.

23. Zusammensetzung nach einem der vorhergehenden Ansprüche, **dadurch gekennzeichnet, dass** die flüssigkristallinen Polymerpartikel in einer Menge von 0,1 bis 99 Gew.-%, bezogen auf das Gesamtgewicht der Zusammensetzung, enthalten sind.

24. Zusammensetzung nach Anspruch 23, **dadurch gekennzeichnet, dass** die flüssigkristallinen Polymerpartikel in einer Menge von 0,1 bis 60 Gew.-% vorliegen.

25. Zusammensetzung nach Anspruch 24, **dadurch gekennzeichnet, dass** die flüssigkristallinen Polymerpartikel in einer Menge von 1 bis 30 Gew.-%, bezogen auf das Gesamtgewicht der Zusammensetzung, enthalten sind.

26. Zusammensetzung nach Anspruch 25, **dadurch gekennzeichnet, dass** die flüssigkristallinen Polymerpartikel in einer Menge von 5 bis 15 Gew.-% des Gesamtgewichts der Zusammensetzung vorliegen.

27. Zusammensetzung nach einem der vorhergehenden Ansprüche, **dadurch gekennzeichnet, dass** die Zusammensetzung ein hydrophiles oder lipophiles kosmetisches Medium enthält.

28. Zusammensetzung nach einem der vorhergehenden Ansprüche, **dadurch gekennzeichnet, dass** sie Wasser oder ein Gemisch von Wasser und einem hydrophilen organischen Lösungsmittel enthält.

29. Zusammensetzung nach einem der vorhergehenden Ansprüche, **dadurch gekennzeichnet, dass** sie eine Fettphase enthält.

30. Zusammensetzung nach Anspruch 29, **dadurch gekennzeichnet, dass** die Zusammensetzung einen Bestandteil enthält, der unter den Ölen, Wachsen, pastösen Fettsubstanzen, Gummis und deren Gemischen ausgewählt ist.

31. Zusammensetzung nach einem der vorhergehenden Ansprüche, **dadurch gekennzeichnet, dass** sie ein organisches Lösungsmittel enthält.

32. Zusammensetzung nach einem der vorhergehenden Ansprüche, **dadurch gekennzeichnet, dass** sie ein filmbildendes Polymer enthält.

33. Zusammensetzung nach Anspruch 32, **dadurch gekennzeichnet, dass** das filmbildende Polymer unter den Vinylpolymeren, Polyurethanen, Polyestern, Polyamiden, Polyharnstoffen und Cellulosepolymeren ausgewählt ist.

34. Zusammensetzung nach einem der Ansprüche 32 oder 33, **dadurch gekennzeichnet, dass** das filmbildende Polymer in einem Trockensubstanzgehalt des Polymers von 0,1 bis 60 Gew.-%, bezogen auf das Gesamtgewicht der Zusammensetzung, enthalten ist.

35. Zusammensetzung nach einem der vorhergehenden Ansprüche, **dadurch gekennzeichnet, dass** sie ein zusätzliches Farbmittel enthält, das von den flüssigkristallinen Polymerpartikeln verschieden ist.

36. Zusammensetzung nach Anspruch 35, **dadurch gekennzeichnet, dass** das zusätzliche Farbmittel unter den Pigmenten, Perlglanzpigmenten, wasserlöslichen oder fettlöslichen Farbstoffen ausgewählt ist.

37. Zusammensetzung nach einem der Ansprüche 35 oder 36, **dadurch gekennzeichnet, dass** das zusätzliche Farbmittel ein Pigment ist, das unter Titandioxid, den Oxiden von Zirconium, Cer, Zink, Eisen und Chrom, Manganviolett, Ultramarinblau, Chromhydrat, Eisenblau, Aluminiumpulver, Kupferpulver, Ruß, den Pigmenten vom Typ D & C und den Lacken auf der Basis von Cochenille-Karmin, Barium, Strontium, Calcium und Aluminium ausgewählt ist.

38. Zusammensetzung nach einem der Ansprüche 36 oder 37, **dadurch gekennzeichnet, dass** die Pigmente in einer Menge von 0,01 bis 15 Gew.-%, bezogen auf das Gesamtgewicht der Zusammensetzung, enthalten sind.

39. Zusammensetzung nach einem der Ansprüche 36 bis 38, **dadurch gekennzeichnet, dass** die Pigmente in einer Menge von 0,02 bis 5 Gew.-%, bezogen auf das Gesamtgewicht der Zusammensetzung, enthalten sind.

40. Zusammensetzung nach einem der Ansprüche 36 bis 39, **dadurch gekennzeichnet, dass** das zusätzliche Farbmittel ein Perlglanzpigment ist, das unter den mit Titan oder Bismutoxidchlorid überzogenen Glimmerpigmenten, mit Eisenoxiden überzogenen Titan-Glimmerpigmenten, mit Eisenblau oder Chromoxid überzogenen Titan-Glimmerpigmenten, mit einem organischen Pigment überzogenen Titan-Glimmerpigmenten und Perlglanzpigmenten auf der Basis von Bismutoxidchlorid ausgewählt ist.

41. Zusammensetzung nach einem der Ansprüche 36 oder 40, **dadurch gekennzeichnet, dass** die Perlglanzpigmente in einer Menge von 0,01 bis 25 Gew.-%, bezogen auf das Gesamtgewicht der Zusammensetzung, enthalten sind.

42. Zusammensetzung nach einem der vorhergehenden Ansprüche, **dadurch gekennzeichnet, dass** sie einen kosmetischen Bestandteil enthält, der unter den Füllstoffen, Vitaminen, Verdickungsmitteln, grenzflächenaktiven Stoffen, Spurenelementen, Hydratisierungsmitteln, reizlindernden Stoffen, Maskierungsmitteln, Parfums, Alkalisierungsmitteln, Ansäuerungsmitteln, Konservierungsmitteln, Antioxidantien, UV-Filtern oder deren Gemischen ausgewählt ist.

43. Zusammensetzung nach einem der vorhergehenden Ansprüche, **dadurch gekennzeichnet, dass** sie als Nagellack, Mascara, Eyeliner, Produkt zum Schminken der Lippen, Make-up, Wagenrouge, Lidschatten, Produkt zum Schminken der Augenbrauen oder Produkt zum Schminken des Körpers vorliegt.

44. Zusammensetzung nach einem der vorhergehenden Ansprüche, **dadurch gekennzeichnet, dass** sie in Form eines Nagellacks vorliegt.

45. Kosmetisches Verfahren zum Schminken von Keratinsubstanzen, **dadurch gekennzeichnet, dass** auf die Keratinsubstanzen eine Zusammensetzung nach einem der Ansprüche 1 bis 44 aufgetragen wird.

46. Kosmetisches Verfahren zum Schminken von Keratinsubstanzen, das umfasst, auf die Keratinsubstanzen eine erste Schicht einer ersten kosmetischen Zusammensetzung aufzutragen, die in einem kosmetisch akzeptablen Medium mindestens ein Farbmittel enthält, wobei die erste Schicht auch als Grundschicht bezeichnet wird, und anschließend auf mindestens einem Teil der ersten Schicht eine zweite Schicht einer zweiten kosmetischen Zusammensetzung aufzubringen, die der Zusammensetzung nach einem der Ansprüche 1 bis 44 entspricht, wobei die erste Zusammensetzung keine flüssigkristallinen Polymerpartikel enthält, wie sie in der zweiten Zusammensetzung enthalten sind.

47. Verfahren nach Anspruch 46, **dadurch gekennzeichnet, dass** die erste Zusammensetzung ein Farbmittel enthält, das unter den Pigmenten, Perlglanzpigmenten, wasserlöslichen oder fettlöslichen Farbstoffen ausgewählt ist.

48. Verfahren nach Anspruch 46 oder 47, **dadurch gekennzeichnet, dass** die erste Zusammensetzung ein filmbildendes Polymer enthält.

49. Verfahren nach einem der Ansprüche 46 und 48, **dadurch gekennzeichnet, dass** die erste Zusammensetzung einen kosmetischen Bestandteil enthält, der unter den Füllstoffen, Vitaminen, Verdickungsmitteln, grenzflächenaktiven Stoffen, Spurenelementen, Hydratisierungsmitteln, reizlindernden Stoffen, Maskierungsmitteln, Parfums, Alkalisierungsmitteln, Ansäuerungsmitteln, Konservierungsmitteln, Antioxidantien, UV-Filtern oder deren Gemischen ausgewählt ist.

50. Verfahren nach einem der Ansprüche 48 bis 49, **dadurch gekennzeichnet, dass** die erste Zusammensetzung als Nagellack, Mascara, Eyeliner, Produkt zum Schminken der Lippen, Make-up, Wangenrouge, Lidschatten, Produkt zum Schminken der Augenbrauen oder Produkt zum Schminken des Körpers vorliegt.

51. Verfahren nach einem der Ansprüche 46 bis 50, **dadurch gekennzeichnet, dass** die erste Zusammensetzung in Form eines Nagellacks vorliegt.

52. Kit zum Schminken umfassend:
- eine erste kosmetische Zusammensetzung, die in einem kosmetisch akzeptablen Medium ein erstes Farbmittel enthält, und
- eine zweite kosmetische Zusammensetzung, die der Zusammensetzung nach einem der Ansprüche 1 bis 44 entspricht,
wobei die erste Zusammensetzung keine flüssigkristallinen Polymerpartikel enthält, wie sie in der zweiten Zusammensetzung enthalten sind,
wobei die erste Zusammensetzung und die zweite Zusammensetzung in unterschiedlichen Behältern konfektioniert sind.

53. Kit zum Schminken nach Anspruch 52, **dadurch gekennzeichnet, dass** die erste Zusammensetzung ein Farbmittel enthält, das unter den Pigmenten, Perlglanzpigmenten, wasserlöslichen oder fettlöslichen Farbstoffen ausgewählt ist.

54. Kit zum Schminken nach Anspruch 52 oder 53, **dadurch gekennzeichnet, dass** die erste Zusammensetzung ein filmbildendes Polymer enthält.

55. Kit zum Schminken nach einem der Ansprüche 52 bis 54, **dadurch gekennzeichnet, dass** die erste Zusammensetzung einen kosmetischen Bestandteil enthält, der unter den Füllstoffen, Vitaminen, Verdickungsmitteln, grenzflächenaktiven Stoffen, Spurenelementen, Hydratisierungsmitteln, reizlindernden Stoffen, Maskierungsmitteln, Parfums, Alkalisierungsmitteln, Ansäuerungsmitteln, Konservierungsmitteln, Antioxidantien, UV-Filtern oder deren Gemischen ausgewählt ist.

56. Kit zum Schminken nach einem der Ansprüche 52 bis 55, **dadurch gekennzeichnet, dass** die erste Zusammensetzung als Nagellack, Mascara, Eyeliner, Produkt zum Schminken der Lippen, Make-up, Wangenrouge, Lidschatten, Produkt zum Schminken der Augenbrauen oder Produkt zum Schminken des Körpers vorliegt.

57. Kit zum Schminken nach einem der Ansprüche 52 bis 56, **dadurch gekennzeichnet, dass** die erste Zusammensetzung als Nagellack vorliegt.

58. Geschminkter Träger, der eine Schminke aufweist, die nach dem Verfahren zum Schminken nach einem der Ansprüche 45 bis 51 erhältlich ist und die auf den Träger aufgetragen wurde, wobei der Träger unter den falschen Nägeln, falschen Wimpern, Haarteilen, Perücken und Punkten oder Patches, die auf der Haut oder den Lippen haften, ausgewählt ist.

## Claims

1. Cosmetic composition comprising, in a cosmetically acceptable medium, particles of liquid crystal polymer which is capable of being obtained by polymerization of a mixture of monomers comprising:
- a) at least one first monomer A of formula (I) Y¹-A¹-M¹-A²-Y²
in which
- i) Y¹ and Y², which are identical or different, represent an acrylate or methacrylate group,
- ii) A¹ and A², which are identical or different, represent a group of formula -CₙH₂ₙ-, in which n is an integer ranging from 1 to 20;
- iii) M¹ denotes a group of general formula (I') -R¹-X¹-R²-X²-R³-X³-R⁴-, in which R¹ and R⁴ denote -O-, R² and R³ denote -COO-, and X¹, X² and X³ are a 1,4-phenylene group, the carbonyl group -CO- respectively of R² and of R³ being bonded respectively to the X¹ group and to the X³ group,
and
- b) at least one chiral second monomer B of formula (II) V¹-W¹-Z-W²-V², in which
- i) V¹ denotes an acrylate or methacrylate group and V² denotes a C₁-C₂₀ alkyl group, a C₁-C₂₀ alkoxy group, a (C₁-C₂₀)alkoxycarbonyl group or an -OH group,
- ii) W¹ represents a divalent group of formula -X'¹-COO-, W² represents a divalent group of formula -O-CO-X'¹-, in which formulae X'¹ denotes a 1,4-phenylene group,
and Z denotes a chiral divalent group resulting from the dianhydrohexite group.

2. Composition according to Claim 1, **characterized in that** V¹ represents an acrylate group.

3. Composition according to Claim 1 or 2, **characterized in that** V² represents a C₁-C₂₀ alkoxy group.

4. Composition according to one of Claims .1 to 3, **characterized in that** Y¹ and Y² represent an acrylate group.

5. Composition according to one of Claims 1 to 4, **characterized in that** A¹ and A², which are identical or different, represent a group of formula -CₙH₂ₙ- in which n is an integer ranging from 2 to 6.

6. Composition according to Claim 5, **characterized in that** n is equal to 4.

7. Composition according to any one of Claims 1 to 6, **characterized in that** V¹ denotes an acrylate group and V² denotes a C₁-C₄ alkoxy group.

8. Composition according to Claim 7, **characterized in that** V² denotes a methoxy group.

9. Composition according to any one of Claims 1 to 8, **characterized in that** Z denotes a divalent radical of formula:

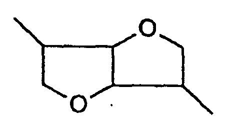

10. Composition according to any one of the preceding claims; **characterized in that** the mixture of monomers comprises from 70 to 99% by weight of monomer A and from 1 to 30% by weight of monomer B with respect to the total weight of monomer A and of monomer B.

11. Composition according to any one of the preceding claims, **characterized in that** the mixture of monomers comprises from 90 to 95% by weight of monomer A and from 5 to 10% by weight of monomer B with respect to the total weight of monomer A and of monomer B.

12. Composition according to any one of the preceding claims, **characterized in that** the liquid crystal polymer exhibits a helical pitch of greater than 450 nm.

13. Composition according to Claim 12, **characterized in that** the liquid crystal polymer exhibits a helical pitch ranging from 455 nm to 5000 nm.

14. Composition according to Claim 12 or 13, **characterized in that** the polymer exhibits a helical pitch ranging from 455 to 1000 nm.

15. Composition according to any one of the preceding claims, **characterized in that** the mixture of monomer A and of monomer B comprises polymerizable groups, at least 90% of which are present in monomers having at least two polymerizable groups, in a concentration ranging from 3.2 to 15 mmol/g.

16. Composition according to any one of the preceding claims, **characterized in that** the monomer A has a weight-average molecular weight ranging from 150 to 800.

17. Composition according to Claim 16, **characterized in that** the monomer has a weight-average molecular weight ranging from 460 to 625.

18. Composition according to any one of the preceding claims, **characterized in that** the monomer B has a weight-average molecular weight ranging from 500 to 1000.

19. Composition according to the preceding claim, **characterized in that** the monomer B has a weight-average molecular weight ranging from 500 to 700.

20. Composition according to any one of the preceding claims, **characterized in that** the particles of liquid crystal polymer are in the form of platelets.

21. Composition according to any one of the preceding claims, **characterized in that** the particles of liquid crystal polymer have a larger size ranging from 1 µm to 3 mm.

22. Composition according to Claim 21, **characterized in that** the particles of liquid crystal polymer have a larger size ranging from 30 µm to 500 µm.

23. Composition according to any one of the preceding claims, **characterized in that** the particles of liquid crystal polymer are present in a content ranging from 0.1% to 99% by weight with respect to the total weight of the composition.

24. Composition according to Claim 23, **characterized in that** the particles of liquid crystal polymer are present in a content ranging from 0.1% to 60% by weight with respect to the total weight of the composition.

25. Composition according to Claim 24, **characterized in that** the particles of liquid crystal polymer are present in a content ranging from 1% to 30% by weight with respect to the total weight of the composition.

26. Composition according to Claim 25, **characterized in that** the particles of liquid crystal polymer are present in a content ranging from 5% to 15% by weight with respect to the total weight of the composition.

27. Composition according to any one of the preceding claims, **characterized in that** the composition comprises a hydrophilic or lipophilic cosmetic medium.

28. Composition according to any one of the preceding claims, **characterized in that** it comprises water or a mixture of water and of hydrophilic organic solvent.

29. Composition according to any one of the preceding claims, **characterized in that** it comprises a fatty phase.

30. Composition according to Claim 29, **characterized in that** the composition comprises an ingredient chosen from oils, waxes, pasty fatty substances, gums and their mixtures.

31. Composition according to any one of the preceding claims, **characterized in that** it comprises an organic solvent.

32. Composition according to any one of the preceding claims, **characterized in that** it comprises a film-forming polymer.

33. Composition according to Claim 32, **characterized in that** the film-forming polymer is chosen from the group formed by vinyl polymers, polyurethanes, polyesters, polyamides, polyureas and cellulose polymers.

34. Composition according to either of Claims 32 and 33, **characterized in that** the film-forming polymer is present in a content on a dry basis of polymer ranging from 0.1% to 60% by weight with respect to the total weight of the composition.

35. Composition according to any one of the preceding claims, **characterized in that** it comprises an additional colouring material different from the particles of the said liquid crystal polymer.

36. Composition according to Claim 35, **characterized in that** the additional colouring material is chosen from the group formed by pigments, pearlescence agents and water-soluble or fat-soluble dyes.

37. Composition according to either of Claims 35 and 36, **characterized in that** the additional colouring material is a pigment chosen from titanium dioxide, zirconium oxides, cerium oxides, zinc oxides, iron oxides, chromium oxides, manganese violet, ultramarine blue, chromium hydrate, ferric blue, aluminium powder, copper powder, carbon black, pigments of D & C type, and lakes based on cochineal carmine or on barium, strontium, calcium or aluminium.

38. Composition according to either of Claims 36 and 37, **characterized in that** the pigments are present in a content ranging from 0.01% to 15% by weight with respect to the total weight of the composition.

39. Composition according to one of Claims 36 to 38, **characterized in that** the pigments are present in a content ranging from 0.02% to 5% by weight with respect to the total weight of the composition.

40. Composition according to any one of Claims 36 to 39, **characterized in that** the additional colouring material is a pearlescence agent chosen from mica covered with titanium oxide or with bismuth oxychloride, titanium oxide-coated mica covered with iron oxides, titanium oxide-coated mica covered with ferric blue or with chromium oxide, titanium oxide-coated mica covered with an organic pigment, or pearlescent pigments based on bismuth oxychloride.

41. Composition according to either of Claims 36 and 40, **characterized in that** the pearlescence agents are present in a content ranging from 0.01% to 25% by weight with respect to the weight of the composition.

42. Composition according to any one of the preceding claims, **characterized in that** it comprises a cosmetic ingredient chosen from the group formed by fillers, vitamins, thickeners, surfactants, trace elements, moisturizers, softeners, sequestering agents, fragrances, basifying or acidifying agents, preservatives, antioxidants, UV screening agents and their mixtures.

43. Composition according to one of the preceding claims, **characterized in that** it is provided in the form of a nail varnish, mascara, eyeliner, make-up product for the lips, foundation, blusher, eye shadow, product for the eyebrows or product for making up the body.

44. Composition according to any one of the preceding claims, **characterized in that** it is provided in the form of a nail varnish.

45. Cosmetic process for making up keratinous substances, **characterized in that** a composition according to any one of Claims 1 to 44 is applied to the keratinous substances.

46. Cosmetic process for making up keratinous substances comprising the application, to the keratinous substances, of a first coat, also known as base coat, of a first cosmetic composition comprising, in a cosmetically acceptable medium, at least one colouring material and then the application, to at least a portion of the said first coat, of a second coat of a second cosmetic composition in accordance with the composition as defined according to any one of Claims 1 to 44,
the first composition not comprising particles of the liquid crystal polymer as are present in the second composition.

47. Process according to Claim 46, **characterized in that** the first composition comprises a colouring material chosen from pigments, pearlescence agents, or water-soluble or fat-soluble dyes.

48. Process according to Claim 46 or 47, **characterized in that** the first composition comprises a film-forming polymer.

49. Process according to any one of Claims 46 to 48, **characterized in that** the first composition comprises a cosmetic ingredient chosen from the group formed by fillers, vitamins, thickening agents, surfactants, trace elements, moisturizers, softeners, sequestering agents, fragrances, basifying or acidifying agents, preservatives, antioxidants, UV screening agents and their mixtures.

50. Process according to any one of Claims 46 to 49, **characterized in that** the first composition is provided in the form of a nail varnish, mascara, eyeliner, make-up product for the lips, foundation, blusher, eye shadow, product for the eyebrows or product for making up the body.

51. Process according to any one of Claims 46 to 50, **characterized in that** the first composition is provided in the form of a nail varnish.

52. Make-up kit comprising:
- a first cosmetic composition comprising, in a cosmetically acceptable medium, a first colouring material, and
- a second cosmetic composition in accordance with the composition as defined according to any one of Claims 1 to 44,
the first composition not comprising particles of the liquid crystal polymer as are present in the second composition,
the first and second compositions being packaged in separate containers.

53. Make-up kit according to Claim 52, **characterized in that** the first composition comprises a colouring material chosen from pigments, pearlescence agents, or water-soluble or fat-soluble dyes.

54. Make-up kit according to Claim 52 or 53, **characterized in that** the first composition comprises a film-forming polymer.

55. Make-up kit according to any one of Claims 52 to 54, **characterized in that** the first composition comprises a cosmetic ingredient chosen from the group formed by fillers, vitamins, thickeners, surfactants, trace elements, moisturizers, softeners, sequestering agents, fragrances, basifying or acidifying agents, preservatives, antioxidants, UV screening agents and their mixtures.

56. Make-up kit according to any one of Claims 52 to 55, **characterized in that** the first composition is provided in the form of a nail varnish, mascara, eyeliner, make-up product for the lips, foundation, blusher, eye shadow, product for the eyebrows or product for making up the body.

57. Make-up kit according to any one of Claims 52 to 56, **characterized in that** the first composition is provided in the form of a nail varnish.

58. Made-up support comprising a make-up capable of being obtained according to the make-up process in accordance with any one of Claims 45 to 51 and applied to the said support, the said support being chosen from false nails, false eyelashes, toupees, wigs, or adherent discs or patches on the skin or lips.
